# EUROPEAN PATENT APPLICATION

(11) **EP 1 174 127 A1**
(43) Date of publication of application: **23.01.2002**
(21) Application number: 00202629.2
(22) Date of filing: 21.07.2000
(51) Int. Cl.: A61K 9/24, A61K 9/36

(54) **Composition for targeted release of an actvie substance and process for producing such a composition**

(71) Applicant: NEDERLANDSE ORGANISATIE VOOR TOEGEPAST-NATUURWETENSCHAPPELIJK ONDERZOEK TNO, 2628 VK Delft (NL)
(72) Inventor: EKHART, Peter Frank, 1078-VJ / Amsterdam (NL); HAAKSMAN, Ingrid Karin, 3823-KG / Amersfoort (NL)
(74) Representative: Jorritsma, Ruurd

(57) **Abstract**

A dosage form for controlled release of an active substance, contains 0.1-80 % by weight of the active substance incorporated in a core comprising at least 50% by weight of a linear glucan, the core being surrounded by a shell comprising at least 50% of a linear glucan, preferably the same glucan. The core and shell material is particularly a dextrin of intermediate molecular weight. The dosage form has zero order release pattern, suitable for administering drug for the treatment of the intestine, and for releasing agrochemicals.

## Description

The invention relates to a dosage form for an active compound which has to be targeted to a specific environment, e.g. a specific part of the digestive tract, where it is released in a controlled fashion.

### Background

There is vast prior art with regard to site-specific delayed release of active agents. Many multi-layer tablet and cylinder systems using multiple hydrophilic polymers based on a swelling/erosion or erosion process have been described. An example is the Geomatrix® multi-layer tablet described by Conte U. et. al. (1992) J. Pharm. Sci., **74**, 926. This tablet contains a core tablet sandwiched between two barrier layers each with its own excipient composition. When a hydrophilic swellable/erodible polymer is used, zero order release kinetics are obtained. The release rate is controlled by diffusion through the barrier layer and reduction of the thickness of this barrier layer, leading to a zero order release kinetics.

Another system was designed by Chidambaram, N. et. al., (1998) J. Control. Rel., **52**, 149. This is a triple layer tablet system consisting of water-insoluble core material including the active compound and two water soluble barrier layers. Linear release is obtained by erosion of the barrier layer, leading to an increasingly exposed core surface. Another example of a delayed release concept is provided by Berliner, D.L. et. al. in US Patent 5,849,327. They use porous microscopic beads containing an active compound plugged into a polysaccharide which can only be degraded by the colon flora. This system is subsequently coated with an enteric material which is resistant during passage through the gastrointestinal tract until the colon is reached, resulting in a colon targeting formula.

A further example is the use of an amylose-based film coating on a tablet described by Basit, A.W. (2000), Pharmaceutical Technology Europe, **12**, (2), 30. This coating can resist the action of the digestive tract to be degraded finally by the colonic flora to deliver its active compound. Other systems known for obtaining delayed release functionality are based on pH sensitivity of the excipient, like the use of copolymers of methacrylic acid described by Ashford, M. et.al. (1993) Int. J. Pharm., **95**, 193.

WO 94/01092 discloses a controlled release dosage form comprising a helical straight-chain amylose excipient. Although this dosage form shows an effective zero-order release rate, it is not possible to delay the start of the release until a desired site or a desired point in time is reached.

A general problem with most prior art systems is the use of multiple excipient components making the tablet intrinsically complex including its safety evaluation. Also the erosion-regulated release tablets can imply the design of a dedicated tablet dimension for each desired dose level in order to obtain a standard release profile. The combination of a programmable delay of a dose level independent controlled release, which is independent of the gastro-intestinal conditions by using a single excipient has not been described so far.

### Description of the invention

It was found according to the invention that a targeted release composition with essentially zero-order release characteristics can be produced using a single excipient material by providing a core containing the active ingredient essentially homogeneously contained in a linear α-glucan (dextrin) excipient and a shell surrounding the core, consisting of the same α-glucan excipient not containing the same active ingredient or containing it in lower concentration only.

An advantage of the present invention is the fact that only one excipient is used. The programmable delay is fully independent from the environment conditions, such as for example enzymatic activity and pH in the intestines, and provides a dose level independent release profile of the active compound. A further advantage is that the dosage form of the invention can be produced with a highly simplified production protocol, a lower risk for allergic reactions or other undesired side-effects when the dosage form is administered to patients and less interactions of the excipient with the drugs enclosed in the composition (or possibly other drugs administered to the patients). The same advantages apply when the dosage form is used for non-therapeutic purposes such as in food supply and feeding, in pest control, in agriculture etc.

The pharmaco-therapeutic advantages of the linear dextrin matrix as novel composition for a delayed release of drugs are the possibility of application for local therapy along the gastrointestinal (GI) tract. The present invention provides a dosage form, which is ideal for local therapy in the GI tract. Technical variations in the production allow the formulation of tablets with different time-release profiles independent of the gastrointestinal conditions. This may result in an oral formulation that decomposes or starts to release in a controlled way, either in the proximal part of the GI tract or in the distal part. This (adjustable) characteristic makes the formulation ideal for local (targeted) therapy to, for example, the colon. A zero-order or an instantaneous release profile can be obtained.

Diseases related to the colon which can be treated with the dosage form of the invention are e.g. inflammatory bowel diseases, Crohn's disease (Morbus Crohn) and Colitis Ulcerosa. Potential candidates to be enclosed in the current controlled-release composition, for treating diseases in the colon include: NSAIDS (non steroidal anti inflammatory drugs, like COX-inhibitors: ibuprofen, diclofenac), mesalazine, sulfasalazine, anti-inflammatory cytokines, corticosteroids or even bioactive peptides and proteins.

An α-glucan is understood to be an essentially straight-chain polysaccharide which is composed of anhydroglucose units which are linked to one another by αbridges, especially through the 1-position and 4-position. Other straight-chain glucans (polysaccharides) can also be used if these are able to assume a helical or similar structure, such as, for example, β-1,3-glucans. Suitable α-glucans include starch fractions and starch derivatives. The α-glucan can, for example, be an amylose, which is a straight chain of *α-*1,4-anhydroglucose units, or analogues with a degree of polymerisation (DP) of the order of 100 - 5,000. Especially suitable α-glucans are linear α-1,4-glucans having a chain length (DP) of 5 or higher. The level of linearity in these α-1,4-glucans is preferably at least 97.5%. The average DP may advantageously be between 5 and 2,000, which does not preclude the presence of longer chains. Useful dextrins include those described in WO 94/01092, WO 96/09815, WO 98/05688 and WO 99/09066. Derivatives which are obtained by debranching branched glucans, in particular amylopectins, are also suitable. To this end the α-1,6 bonds of amylopectin are broken, preferably enzymatically (see Kobayashi, S. et al., *Cereal Chem.* **63**, 71-74 (1968) and NL patents 160,615 and 165,500) with the formation of amylodextrin, a straight-chain dextrin. Depending on the starch source, these amylodextrins usually show multimodal chain length distributions with average peak chain lengths (DP) ranging from 5 to about 700, whereas individual amylodextrin molecules may range from DP 3 to more than 1,000 (Takeda et al. *Carbohydr*. *Res.* 246 (1993) 273-281). Depending on production conditions and presence of complex forming agents, amylodextrin, may contain single or double helices with 3 to 8 anhydroglucose units per turn.

The excipient material, either in the core or in the shell or both, can contain other matrix materials, fillers and auxiliaries, including water. The presence of such other materials up to a content of, for example, 25 % or even 50% by weight does not interfere with the release performance of the dosage form. Other matrix materials may include other polysaccharides, such as starch, cellulose and derivatives, fructans and the like, as well as synthetic, degradable polymers. Suitable auxiliaries are the auxiliaries known per se for compositions of active substances, such as lubricants, for example magnesium stearate, auxiliary solvents, pH regulators, preservatives, disintegrating agents, colorants, flavourings and the like. Auxiliaries which modify the release pattern of the active substance from the matrix, such as auxiliaries which in themselves are inactive, for example lactose, or active auxiliaries such as α-amylase, which can help the matrix material to disintegrate from inside out, may also be present.

The active substance can be present in core material in virtually any desired concentration. In general, the amount of active substance can make up, for example, 0.1 - 80 % by weight of the composition, partly depending on the desired dosage. More particularly, the amount is between 0.5 and 50 % by weight. Within this range, a release rate is obtained which is independent of the remaining amount of active substance, that is to say which has a zero order release curve.

The shell material does not contain the active substance contained in the core material, or only at a lower level, i.e. at least two times lower than in the core material. Low levels of active substance in the shell can be useful in cases where rapid release is required but where "dumping", i.e. excessive release over a short period, should be avoided. Alternatively a second active material having a different effect and a different target environment can be present in the shell material.

The rate of release of the composition according to the invention can be adjusted by varying one or more of the following parameters: properties of the shell material, active substance concentration in the core material, dosage unit form, in particular the surface area/capacity ratio, force under which the composition is pressed, presence of disintegration-promoting agents, for example lactose or α-amylase, or disintegration-retarding agents, such as a coating or an inert filler.

The active substance can be of diverse natures. In addition to the use for administration in the colon as described above, the active substance can be suited for oral, rectal, vaginal or transdermal administration, diagnostic agents, feedstuffs or conditioning agents, flavourings, manure or nutrients to be added to water or soil, preservatives, vaccine substances, hormones, genetic material, control agents, attractants, growth promoters and the like. The active substances can be either organic or inorganic. Mixtures of active substance can also be administered by means of the composition according to the invention. Release can take place in an aqueous medium, such as the gastrointestinal tract of animals, or in plants or in the soil, but also into the air. The dosage forms of the invention are particularly suitable for releasing relatively small active substances, e.g. having a molecular weight of less than 1,500 daltons. Larger molecules can also be released effectively, but may require the presence of a disintegrating aid such as lactose or an α-amylase, as described in WO 94/01091.

### Example

### Preparation of linear dextrins

A hundred g of waxy maize starch or isolated potato amylopectin was added to 1750 ml of a 5 mM sodium acetate buffer at 100°C with stirring to provide a 6 wt.% gelatinised starch solution with a pH of 5.2. At 55°C 5 ml of Promozym 400 L (400 PUN/ml) was added to this solution and reacted overnight. Subsequently the enzyme was deactivated by heating to 100°C for 5 minutes. The denatured protein was filtered off using a P2 glassfrit. To the filtered solution, 550 ml of ethanol was added and the solution was cooled to room temperature by placing the solution at 4°C overnight. The precipitate was filtered and washed with 100% ethanol and dried at room temperature. The linear dextrins were stored at 65% relative humidity. The linear dextrins from potato or maize origin had a mean DP of 24 and 21, respectively, and were stored at 65% relative humidity.

### Production of controlled-release tablets

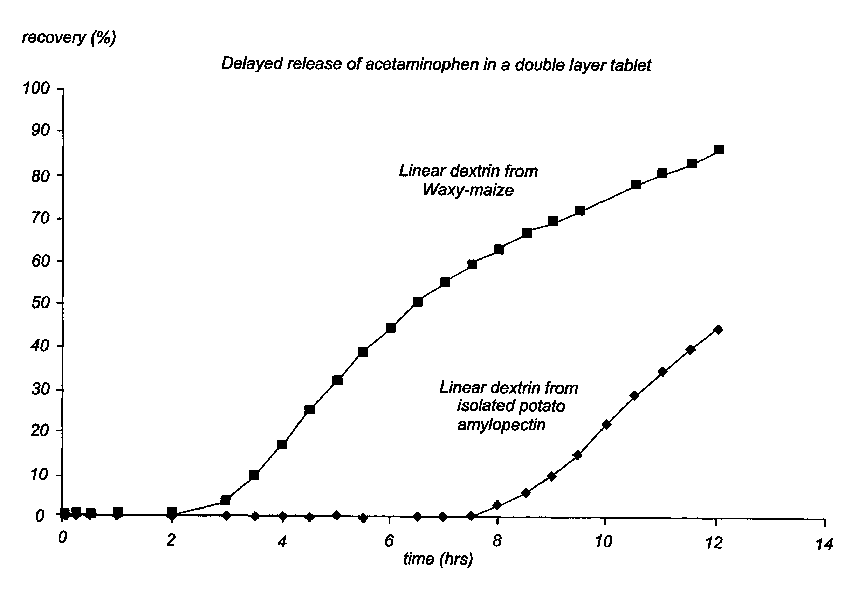

Double-layered tablets were produced as follows: Acetoaminophen (30%) was mixed with amylodextrins based on either waxy maize starch or isolated potato amylopectin as described above. Tablets of 40 mg having a diameter of 3 mm were pressed. After addition of further amylodextrin without acetaminophen, these tablets were pressed to tablets of 400 mg having a diameter of 9 mm. The release pattern of acetaminophen from these double tablets was determined in a pharmacopoeia paddle arrangement. The results are shown in figure 1.

## Claims

1. A dosage form for controlled release of an active substance, the active substance being incorporated in a matrix material, **characterised in that** the active substance is incorporated in a core comprising at least 50% by weight of an essentially linear α-glucan, the core being surrounded by a shell comprising at least 50% by weight of an essentially linear α-glucan and containing a concentration of the active substance of less than 50% of the concentration in the core.

2. A dosage form according to Claim 1, in which the active substance is present in an amount of 0.1-80 % by weight of the core, preferably in an amount of 0.5-50 % by weight of the core.

3. A dosage form according to Claim 1 or 2, in which the linear α-glucan contains at least 97.5% of α-1,4-linked anhydroglucose units having a degree of polymerisation (DP) of 3 and higher.

4. A dosage according to one of Claims 1-3, in which the essentially linear α-glucan in the core is of the same type as the essentially linear α-glucan in the shell.

5. A dosage according to one of Claims 1-4, in which the active substance is a medicament.

6. A dosage form according to Claim 5, in which the active substance is intended for treatment of the jejunum or colon.

7. A dosage form according to one of Claims 1-4, in which the active substance is an agrochemical.

8. A dosage form according to one of Claims 1-7, in which a second active substance is present in the shell.

9. A dosage form according to any one of the preceding claims, which is a tablet.

10. A dosage form according to any one of the preceding claims, which furthermore contains a disintegrating aid.

11. A process for producing a dosage form according to one of the preceding claims, **characterised in that** an α-glucan is granulated and mixed, before or after granulating, with an active substance and the granulated mixture is brought into a shaped form, and said shaped together with a further amount of the α-glucan is brought into a further shaped form.
